# EUROPEAN PATENT SPECIFICATION

(11) **EP 2 928 380 B1**
(45) Date of publication and mention of the grant of the patent: **01.07.2020**
(21) Application number: 13861256.9
(22) Date of filing: 04.12.2013
(51) Int. Cl.: A61B 8/12, A61L 29/04, A61L 29/14, A61M 25/00

(54) **REINFORCED CATHETER TRANSITION WITH FLEXIBLE TIP PORTION**
VERSTÄRKTER KATHETERÜBERGANG MIT FLEXIBLEM SPITZENTEIL
TRANSITION DE CATHÉTER RENFORCÉE AVEC PARTIE DE POINTE FLEXIBLE

(30) Priority: 06.12.2012 US 201261734286 P
(43) Date of publication of application: 14.10.2015
(73) Proprietor: Volcano Corporation, San Diego, CA 92130 (US)
(72) Inventor: VAN HOVEN, Dylan, Oceanside, California 92057 (US)
(74) Representative: de Haan, Poul Erik
(86) International application number: PCT/US2013/073081
(87) International publication number: WO 2014/089187

(56) References cited:
- US-A1- 2003 036 729
- US-A1- 2005 131 445
- US-A1- 2006 129 178
- US-A1- 2008 262 470
- US-A1- 2008 262 470
- US-A1- 2009 264 826
- US-A1- 2010 094 259
- US-A1- 2011 208 164

## Description

### TECHNICAL FIELD

The present disclosure relates generally to catheters for navigating through the human vasculature, and in particular, to improved catheter tip designs and reinforced sections of the catheter.

### BACKGROUND

Intravascular ultrasound (IVUS) imaging is widely used in interventional cardiology as a diagnostic tool for assessing a vessel, such as an artery, within the human body to determine the need for treatment, to guide intervention, and/or to assess its effectiveness. An IVUS imaging system uses ultrasound echoes to form a cross-sectional image of the vessel of interest. Typically, IVUS imaging uses a transducer carried by an IVUS catheter that both emits ultrasound signals (waves) and receives the reflected ultrasound signals. The emitted ultrasound signals (often referred to as ultrasound pulses) pass easily through most tissues and blood, but they are partially reflected by discontinuities arising from tissue structures (such as the various layers of the vessel wall), red blood cells, and other features of interest. The IVUS imaging system, which is connected to the IVUS catheter by way of a patient interface module, processes the received ultrasound signals (often referred to as ultrasound echoes) to produce a cross-sectional image of the vessel where the IVUS catheter is located.

US 2005/131445 A1 discloses distal tip designs for catheter, wherein distal tip material is positioned about an inner shaft and which is used as a tie layer for thermally bonding two incompatible materials together, such as a waist portion of a balloon to the inner shaft.

US 2008/262470 A1 discloses a balloon catheter having a soft distal tip member having a non-tacky inner layer material and a soft flexible outer layer material, with both materials being readily thermally bondable to the catheter balloon.

US 2011/208164 A1 discloses a device having a first sheath with a proximal end portion and a distal end portion defining a passageway therebetween, wherein the distal end portion has melt bonding material. A second sheath has a first end portion and a second end portion defining a channel therebetween, wherein the second end portion has melt bonding material. An outer sleeve body comprising melt-bonding material operatively couples to the first sheath distal end portion and to the second sheath second end portion.

Short guide wire lumen rapid exchange (RX) catheter designs or "monorail" designs generally employ a much shorter guide wire lumen at the distal end of the catheter, typically in the range from about 1 cm to 4 cm. The transducer may then be disposed axially spaced but close to the guide wire lumen, allowing a reduction in the total cross-sectional area of the catheter. Among the difficulties sometimes encountered with short tipped guide wire catheters is the possibility that the distal end or tip of the catheter may kink as it is advanced through the patient's vasculature. The unwanted bending may occur in the region of a guide wire side port, irrigation port, or any unsupported region distal region of the imaging system sheath. Kinking is the result of a deformation of the distal tip and usually is characterized by a sharp deformation or point bend of the very distal section of the catheter. Such a deformation may result from attempting to pass the distal tip through a very tortuous vascular section. Parts of the catheter may also kink or bend back upon itself in a condition referred to as prolapse. Thereafter, the catheter may return to its original shape, or it may remain permanently deformed if, during the bending, catheter material is bent beyond its elastic limit.

Once the catheter has been kinked, the performance of the catheter is substantially degraded. Higher friction will be encountered at the location of the kink, adversely affecting torque transmission, as well as making it more difficult to advance the catheter over the guide wire.

The transition between the proximal section and the distal section of the catheter should also provide a good transition in flexibility from the relatively stiff proximal section to the relatively flexible distal section to facilitate tracking the catheter within the patient's tortuous vasculature. One difficulty has been that catheter junctions often result in a lump, step, or other surface irregularity in the bond junction.

Therefore, a need exists for catheters that are resistant to kinking or prolapse when introduced through tortuous regions of blood vessels.

### SUMMARY

According to the present invention, there is provided a sheath for an intravascular probe for insertion into a vasculature, in accordance with claim 1.

The present disclosure provides improved structural arrangements for the distal portion of intravascular devices, including intravascular imaging devices. In particular, the constructions of the present disclosure result in intravascular devices having improved handling characteristics due to the combination of flexibility and resistance to kinking or prolapse when introduced through tortuous regions of blood vessels.

The present disclosure describes various examples of a distal tip construction for use in intravascular ultrasound (IVUS) imaging. An exemplary tip construction includes three layers. The first layer is an inner layer adjacent to a guide wire lumen, the second layer is a middle layer adjacent the inner layer, and the third layer is an outer layer adjacent the middle layer. The first layer typically includes a lubricious polymer to promote low friction and ease of tracking over the guide wire. The second layer generally includes a polymer that adheres well to both the inner and outer layers. The third layer usually includes a polymer that is flexible and that can also be coated with a hydrophilic coating. This construction provides low friction and good guide wire movement, along with reduced friction between the catheter and blood vessel wall.

In other examples, the sheath includes a flexible portion having a lumen for receiving an ultrasound probe and a distal portion that includes a flexible tip. The tip defines a guide wire lumen having a distal guide wire opening and a proximal guide wire opening through a sidewalk. An area between the proximal guide wire entry opening and the flexible proximal portion is supported to prevent prolapse. In some examples, the area is supported by a tube, which may comprise a polyimide and/or stainless steel. In alternative examples, the tube includes an inner lumen in communication with the ultrasound lumen and a flush hole through the tube. The sheath provides a flexible device that is locally reinforced between the proximal guide wire entry opening and ultrasound probe so that the monorail is flexible, tracks well, and does not prolapse. The additional support prevents localized kinking.

Both the foregoing general description and the following detailed description are exemplary and explanatory in nature and are intended to provide an understanding of the present disclosure without limiting the scope of the present disclosure. In that regard, additional aspects, features, and advantages of the present disclosure will become apparent to one skilled in the art from the following detailed description.

### BRIEF DESCRIPTIONS OF THE DRAWINGS

Aspects of the present disclosure are best understood from the following detailed description when read with the accompanying figures. It is emphasized that, in accordance with the standard practice in the industry, various features are not drawn to scale. In fact, the dimensions of the various features may be arbitrarily increased or reduced for clarity of discussion. In addition, the present disclosure may repeat reference numerals and/or letters in the various examples. This repetition is for the purpose of simplicity and clarity and does not in itself dictate a relationship between the various embodiments and/or configurations discussed.
FIG. 1 is an illustration of an intravascular ultrasound (IVUS) imaging catheter according to various aspects of the present disclosure.
FIG. 2 is a stylized top view of a distal portion of a sheath for a rotational imaging system.
FIG. 3 is a diagrammatic cross-sectional side view of the sheath of FIG. 2 taken along line 3-3.
FIG. 4 illustrates a subassembly of catheter shaft portions to be bonded according to various aspects of the present disclosure.
FIG. 5 illustrates a transition junction between sheath sections of different diameters.

### DETAILED DESCRIPTION

For the purposes of promoting an understanding of the principles of the present disclosure, reference will now be made to the embodiments illustrated in the drawings, and specific language will be used to describe the same.

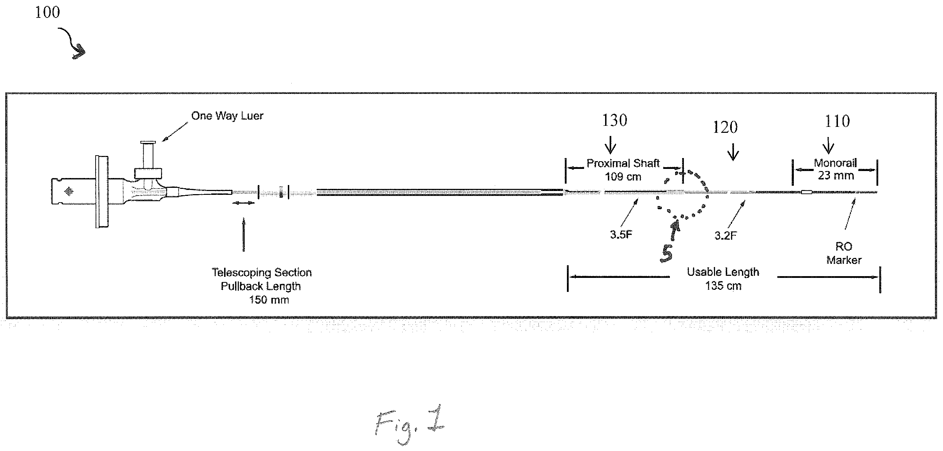

Referring specifically to FIG. 1, a rotational intravascular probe or catheter 100 for insertion into a patient for diagnostic imaging is shown. In some embodiments, the IVUS probe 100 is similar to a Revolution® Rotational IVUS Imaging Catheter available from Volcano Corporation and/or rotational IVUS catheters disclosed in U.S. Patent No. 5,243,988 and U.S. Patent No. 5,546,948. The probe 100 includes an elongated, flexible catheter sheath having a flexible proximal portion 130, a distal portion 110 shaped and configured for insertion into a lumen of a blood vessel, and a transition portion 120 that couples the proximal portion and the distal portion. The probe 100 is flexible such that it can adapt to the curvature of the blood vessel during use. Generally, the probe 100 may be configured to take on any desired straight or arcuate profile when in use.

The probe 100 comprises a sheath and a transducer shaft surrounded by the sheath. The transducer shaft is flushed with a sterile fluid, such as saline, within the catheter body. The fluid serves to eliminate the presence of air pockets or bubbles around the transducer shaft that adversely affect image quality. The fluid can also act as a lubricant.

The distal portion 110 of the sheath is inserted into a patient during the operation of the probe 100. The distal portion 110 includes a short tip. With short tips, only the very distal end of the catheter follows the guide wire, while the remainder of the catheter is left to move as needed within a blood vessel. The short tip in illustrated embodiment is about 22-23 mm compared to about 22 cm for most other long tipped coronary RX catheters. In one embodiment, the tip is 23 mm.

The transition portion 120 of the sheath is typically only about 1-3 mm, but is typically not supported by a guide wire like the tip, or by the transducer shaft like the proximal portion 130. In one embodiment, the transition portion 120 is only about 3 mm in length and has a diameter of about 1.07 mm (3.2 French (F)) or about 1 mm. Thus, in prior designs this unsupported area is prone to kinking or prolapse.

The proximal portion 130 of the sheath and the proximal end portion of the transducer shaft are connected to an interface module. The rotation of the transducer shaft within the catheter body is controlled by the interface module, which provides a plurality of user interface controls that can be manipulated by a user. The interface module can receive, analyze, and/or display information received through the transducer shaft.

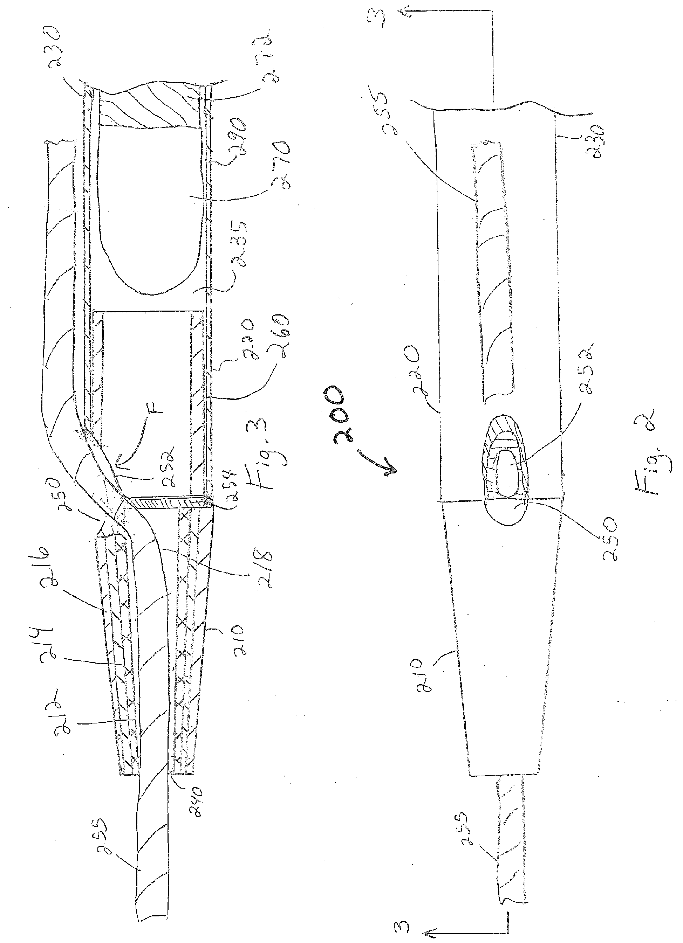

The usable length of the probe 100 (the portion that can be inserted into a patient) can be any suitable length and can be varied depending upon the application. The overall dimensions of the catheter will depend on use, with the length varying widely, typically being between about 40 cm and 150 cm, usually being between about 40 cm and 120 cm for peripheral catheters and being between about 110 cm and 150 cm for coronary catheters. The diameter of the catheter body may also vary widely, with the diameter of the distal portion 110 typically being between about 0.67 mm (2F) and 1.33 mm (4F), and the diameter of the proximal portion 130 typically being about 1 mm (3F) and 2 mm (6F). In an exemplary embodiment, the diameter of the proximal portion 130 is 1.17 mm (3.5 F). Turning now to FIGS. 2 and 3, illustrated is a portion of a sheath 200 showing a stylized portion of the distal tip 210 and the reinforced transition region 220 according to the present disclosure. FIG. 2 is a top view, while FIG. 3 is cross-sectional side view taken along line 3-3 of FIG. 2. For purposes of illustration, the sheath 200 is axially compressed. The sheath 200 employs a RX design, whereby a guide wire 255 enters the sheath 200 less than about 0.3 cm from the distal end or tip to allow an ultrasound probe 270 to be placed as close to the tip as possible. In use, the sheath 200 may be placed on guide wire 255 by threading the guide wire 255 through the distal guide wire opening 240, then the guide wire lumen 218, and finally the proximal guide wire opening 250. As shown in FIGS. 2 and 3, the proximal guide wire exit opening 250 extends through a sidewall of the sheath 200. The catheter body can then be then advanced along the guide wire 255 until the sheath 200 lies within the region of interest.

In one embodiment, proximal guide wire exit opening 250 includes a flush hole 252 to allow saline to flow out of the ultrasound probe lumen 235 in the direction of arrow F. In an alternative embodiment, a separate flush hole (not shown) is formed through support tube 260 and outer tube 290 opposite the guide wire exit opening.

The ultrasound probe lumen 235 may extend from the proximal end of the sheath 200 to the distal tip thereof, but will usually be terminated before reaching the distal tip. Thus, the guide wire lumen 218 may be disposed at least partially adjacent to the ultrasound probe lumen 235.

The distal tip portion 210 is coupled to the transition portion 220 and the proximal portion 230 by adhesive bonding 254. As illustrated, transition portion 220 and proximal portion 230 include the outer tube 290 that defines the ultrasound probe lumen 235. Within the transition portion 220 is support tube 260 to help support the transition portion 220 during use. The support tube 260 provides stiffness and controlled bending of the sheath 200 in the region proximal the proximal guide wire exit opening 250 and distal the ultrasound probe 270. As shown in FIG. 3, the ultrasound probe 270 extends within the ultrasound probe lumen but does not extend into the support tube 260.

The ultrasound probe 270 is located at an end of a flexible transducer drive shaft that spins inside the sheath 200 inserted into the vessel of interest. The ultrasound probe 270 is usually oriented such that the ultrasound signals propagate generally perpendicular to an axis of the catheter. In the typical rotational catheter, the fluid-filled (e.g., saline-filled) sheath 290 protects the vessel tissue from the spinning probe and shaft while permitting ultrasound signals to freely propagate from the probe into the tissue and back. As the shaft rotates (for example, at 30 revolutions per second), the probe is periodically excited with a high voltage pulse to emit a short burst of ultrasound. The ultrasound signals are emitted from the probe, through the fluid-filled sheath and sheath wall, in a direction generally perpendicular to an axis of rotation of the shaft. The same probe then listens for returning ultrasound signals reflected from various tissue structures, and the imaging system assembles a two dimensional image of the vessel cross-section from a sequence of several hundred of these ultrasound pulse/echo acquisition sequences occurring during a single revolution of the probe.

Turning back to FIG. 3, the distal portion 210 includes a flexible distal tip having a multi-layer construction. The distal tip includes three layers: an inner layer 212 adjacent the guide wire lumen 218, a middle layer 214 adjacent the inner layer 212, and an outer layer 216 adjacent the middle layer 214. Flexibility is needed to make tortuous turns in the vasculature while following the guide wire 255, but rigidity is also needed to allow the tip to track along the guide wire.

In one embodiment, the length of the distal tip 210 is about 22 mm. In an alternative embodiment, the length of the tip is less than about 3 cm.

The inner layer 212 includes a lubricious polymer that slides easily over guide wire 255. In an exemplary embodiment, the lubricious polymer includes a high-density polyethylene to promote low friction and ease of tracking over the guide wire 255.

The middle layer 214 includes a polymer that adheres to the inner layer 212 and the outer layer 216. In an exemplary embodiment, the polymer includes a functionalized or maleic anhydride grafted polyethylene.

The outer layer 216 includes a flexible polymer. In an exemplary embodiment, the flexible polymer includes a polyether block amide and/or polyamide. The flexible polymer can make tight or tortuous bends in the anatomy. In a further aspect, the outer layer 216 can be coated with a polymeric hydrophilic coating to reduce tracking friction against vessel walls in the anatomy.

The transition portion 220 includes support tube 260. The support tube 260 is located in an area between the proximal guide wire opening 250 and the flexible proximal portion 230. The support tube 260 may be positioned within a distal extremity of the lumen 235. The support tube 260 is formed of any material suitable to reinforce the transition portion 220 so that the transition portion 220 is supported and does not prolapse. In an exemplary embodiment, the support tube 260 includes polyimide and/or stainless steel. In an embodiment, the support tube 260 includes an inner lumen in communication with the ultrasound probe lumen 235 and a flush hole 252 through at least a portion of the support tube 260. Without limitation to support members having alternative lengths, in the illustrated embodiment, the support member 260 has a length between 1-3 mm. In a further aspect, the support member 260 has a length of approximately 2 mm.

The sheath 200 of the present disclosure has excellent ability to track within the patient's tortuous vasculature due to the improved design of the distal tip portion 210 and the reinforced transition portion 220.

The outer sheath 290 may be formed from a single tubular member that extends the entire distance from the proximal portion 130 to the distal portion 110 or may be formed from two or more tubular members that are joined together. The two tubular members may be joined together so that they share a common inner lumen. As shown in FIG. 1 at detail 5, the outer sheath 290 transitions from a distal diameter of 1.07 mm (3.2F) to a proximal diameter of 1.17 mm (3.5 F).

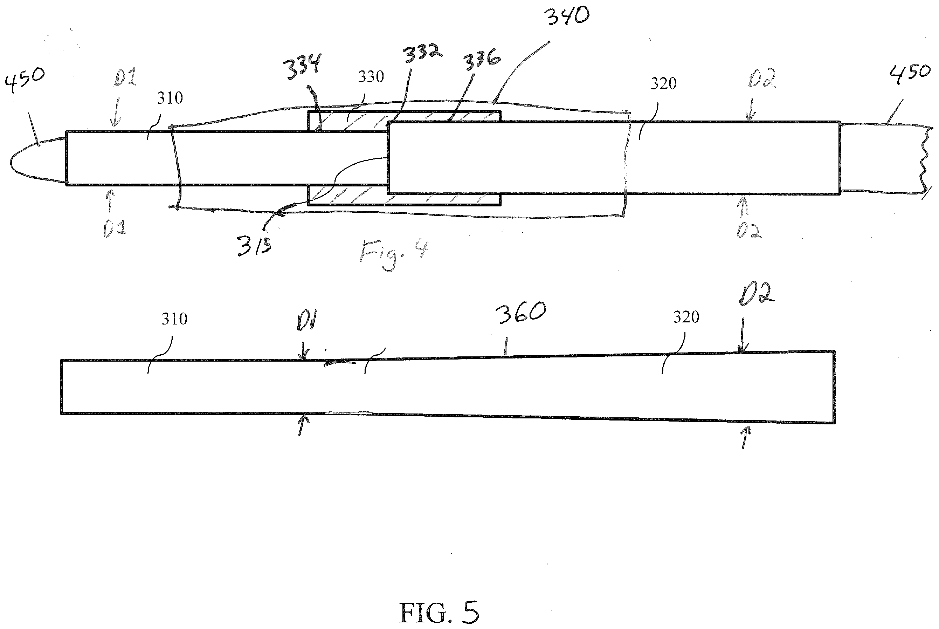

Referring now to FIG. 4, shown is a subassembly of catheter shaft portions to be bonded. The subassembly is formed by positioning one end of a first catheter shaft portion 310 into an end of a second catheter shaft portion 320. The first catheter shaft portion 310 has an outer diameter D1 of approximately 1.07 mm (3.2F) that is different than the outer diameter D2 of approximately 1.17 mm (3.5F) of the second catheter shaft portion 320. A mandrel 350 is positioned in the inner lumen to keep the inner lumen open during the fusing of the first catheter shaft portion 310 and second catheter shaft portion 320. A sleeve 330 is placed over a junction 315 of the two shaft portions 310, 320. In an embodiment, the sleeve 330 includes the same material as one or both of the two shaft portions 310, 320. In an exemplary embodiment, the sleeve 430 includes a nylon and/or polyether block amide. The sleeve includes a shoulder 332 separating a first portion 334 having a first diameter substantially matching D1 and a second portion 336 having a diameter substantially matching D2. A heat shrink thermoplastic sleeve 340 is placed over the sleeve 330 and the junction 315 to create final subassembly.

Bonding of the subassembly is completed by applying heat to the subassembly melt the catheter portions and sleeve. During heating, the heat shrink thermoplastic sleeve 340 shrinks and constrains a flow of the sleeve material 330 and catheter materials at the junction 315. During the heating, the sleeve material 330 melts and fuses the two shaft portions 310, 320 together. The sleeve material 330 flows and fills in around the junction 315 to form a transition zone 360. After cooling, the heat shrink thermoplastic sleeve 340 is removed.

Referring now to FIG. 5, the heat shrink thermoplastic sleeve 340 created a smooth and long transition zone 360 from the first shaft portion 310 to the second shaft portion 320 over the junction 315. In addition, the heat shrink thermoplastic sleeve 440 results in a stronger junction 415 compared to a method that does not use sleeve 440. The use of a thermoplastic sleeve helps to strengthen and improve the thermally bonded catheter junctions by filling in surface irregularities and providing a smooth transition from one shaft to the next over the entire length of the bond junction. This smooth transition presents an atraumatic surface to tissue as the sheath is advanced within the body.

Persons skilled in the art will recognize that the devices and methods described above can be modified in various ways.

## Claims

1. A sheath (200) for an intravascular probe for insertion into a vasculature, the sheath comprising:
a flexible sheath (290) having an ultrasound probe lumen (235) for receiving a sensing probe;
a distal tip (210) attached to a distal extremity of the flexible sheath and defining a guide wire lumen (218) having a distal guide wire opening (240) and a proximal guidewire opening (250) extending through a sidewall, wherein the distal tip includes an inner layer (212) adjacent the guide wire lumen, a middle layer (214) adjacent the inner layer, an outer layer (216) adjacent the middle layer;
a support member (260) positioned within at least the distal extremity of the flexible sheath adjacent the distal tip and forming a reinforced transition region (220), wherein the support member is configured to prevent collapse of the distal extremity of the sheath during advancement along the guidewire; and
the proximal guidewire opening (250) includes a flush hole (252) configured to allow saline to flow out of the ultrasound proble lumen (235).

2. The sheath of claim 1, wherein the inner layer comprises a lubricious polymer, the middle layer comprises a polymer that adheres to the inner and outer layers, and the outer layer comprises a flexible polymer.

3. The sheath of claim 2, wherein the lubricious polymer comprises a high-density polyethylene, the polymer that adheres to the inner and outer layers comprises a functionalized or maleic anhydride grafted polyethylene, and the flexible polymer comprises a polyether block amide and/or polyamide.

4. The sheath of claim 1, wherein the distal tip further comprises a hydrophilic coating adjacent the outer layer.

5. The sheath of claim 1, wherein the length of the distal tip is about 22 mm.

6. The sheath of claim 1, wherein the length of the distal tip is less than about 3 cm.

7. The sheath of claim 1, wherein the support member is a tube.

8. The sheath of claim 7, wherein the tube comprises an inner lumen in communication with an ultrasound lumen and a flush hole through the tube.

## Patentansprüche

1. Hülle (200) für eine intravaskuläre Sonde zum Einführen in ein Gefäßsystem, wobei die Hülle umfasst:
eine flexible Hülle (290) mit einem Ultraschallsondenlumen (235) zum Aufnehmen einer Erfassungssonde;
eine distale Spitze (210), die an einem distalen Ende der flexiblen Hülle angebracht ist, und ein Führungsdrahtlumen (218) mit einer distalen Führungsdrahtöffnung (240) und einer proximalen Führungsdrahtöffnung (250) definiert, die sich durch eine Seitenwand erstreckt, wobei die distale Spitze eine innere Schicht (212) neben dem Führungsdrahtlumen, eine mittlere Schicht (214) neben der inneren Schicht, eine äußere Schicht (216) neben der mittleren Schicht umfasst;
ein Stützelement (260), das innerhalb mindestens des distalen Endes der flexiblen Hülle neben der distalen Spitze positioniert ist, und einen verstärkten Übergangsbereich (220) bildet, wobei das Stützelement so konfiguriert ist, dass ein Zusammenfallen des distalen Endes der Hülle während des Vorschiebens entlang der Führungsdraht verhindert wird; und
die proximale Führungsdrahtöffnung (250) ein Spülloch (252) enthält, das so konfiguriert ist, dass Kochsalzlösung aus dem Ultraschallsondenlumen (235) herausfließen kann.

2. Hülle nach Anspruch 1, wobei die innere Schicht ein Schmierpolymer umfasst, die mittlere Schicht ein Polymer umfasst, das an der inneren und äußeren Schicht haftet, und die äußere Schicht ein flexibles Polymer umfasst.

3. Hülle nach Anspruch 2, wobei das Schmierpolymer ein Polyethylen mit hoher Dichte umfasst, das an der inneren und äußeren Schicht anhaftende Polymer ein funktionalisiertes oder mit Maleinsäureanhydrid gepfropftes Polyethylen umfasst und das flexible Polymer ein Polyetherblockamid und/oder Polyamid umfasst.

4. Hülle nach Anspruch 1, wobei die distale Spitze ferner eine hydrophile Beschichtung neben der äußeren Schicht aufweist.

5. Hülle nach Anspruch 1, wobei die Länge der distalen Spitze etwa 22 mm beträgt.

6. Hülle nach Anspruch 1, wobei die Länge der distalen Spitze weniger als etwa 3 cm beträgt.

7. Hülle nach Anspruch 1, wobei das Stützelement ein Rohr ist.

8. Hülle nach Anspruch 7, wobei das Rohr ein inneres Lumen in Verbindung mit einem Ultraschalllumen und ein Spülloch durch das Rohr umfasst.

## Revendications

1. Gaine (200) pour une sonde intravasculaire à insérer dans un système vasculaire, la gaine comprenant:
une gaine flexible (290) ayant un lumen de sonde à ultrasons (235) pour recevoir une sonde de détection;
une pointe distale (210) fixée à une extrémité distale de la gaine flexible et définissant un lumen de fil de guidage (218) ayant une ouverture de fil de guidage distale (240) et une ouverture de fil de guidage proximale (250) s'étendant à travers une paroi latérale, où la pointe distale comprend une couche intérieure (212) adjacente au lumen du fil de guidage, une couche médiane (214) adjacente à la couche intérieure, une couche extérieure (216) adjacente à la couche médiane;
un élément de support (260) positionné à l'intérieur d'au moins l'extrémité distale de la gaine flexible adjacente à la pointe distale et formant une région de transition renforcée (220), où l'élément de support est configuré pour empêcher l'affaissement de l'extrémité distale de la gaine pendant l'avancement le long du fil de guidage; et
l'ouverture proximale du fil de guidage (250) comprend un trou de rinçage (252) configuré pour permettre au sérum physiologique de s'écouler hors du lumen de sonde à ultrasons (235).

2. Gaine selon la revendication 1, dans laquelle la couche intérieure comprend un polymère lubrifiant, la couche médiane comprend un polymère qui adhère aux couches intérieure et extérieure, et la couche extérieure comprend un polymère flexible.

3. Gaine selon la revendication 2, dans laquelle le polymère lubrifiant comprend un polyéthylène haute densité, le polymère qui adhère aux couches intérieure et extérieure comprend un polyéthylène greffé fonctionnalisé ou d'anhydride maléique, et le polymère flexible comprend un polyamide et/ou amide à blocs polyéthers.

4. Gaine selon la revendication 1, dans laquelle la pointe distale comprend en outre un revêtement hydrophile adjacent à la couche externe.

5. Gaine selon la revendication 1, dans laquelle la longueur de la pointe distale est d'environ 22 mm.

6. Gaine selon la revendication 1, dans laquelle la longueur de la pointe distale est inférieure à environ 3 cm.

7. Gaine selon la revendication 1, dans laquelle l'élément de support est un tube.

8. Gaine selon la revendication 7, dans laquelle le tube comprend un lumen intérieur en communication avec un lumen de ultrasons et un trou de rinçage à travers le tube.
